# FASCICULE DE BREVET EUROPEEN

(11) **EP 1 185 713 B1**
(45) Date de publication et mention de la délivrance du brevet: **02.05.2007**
(21) Numéro de dépôt: 00949555.7
(22) Date de dépôt: 21.06.2000
(51) Int. Cl.: C12Q 1/70

(54) **Procédé de recherche d'une résistance aux anti-protéases d'une souche du virus VIH-2 provenant d'un échantillon biologique prélevé chez un patient**
Screeningverfahren auf Anti-Protease Resistenz HIV-2-haltiger Zellen, ausgehend von biologischen Patientenproben
Search method for resistance to anti-proteases of a strain of the HIV 2 virus from a biological sample taken from a patient

(30) Priorité: 21.06.1999 FR 9907855
(43) Date de publication de la demande: 13.03.2002
(73) Titulaire: BIO MERIEUX, 69280 Marcy l'Etoile (FR); ASSISTANCE PUBLIQUE, HOPITAUX DE PARIS, 75004 Paris (FR)
(72) Inventeur: TELLES, Jean-No¬l, F-75017 Paris (FR); BRUN-VEZINET, Françoise, F-75005 Paris (FR); DESCAMPS, Diane, F-75015 Paris (FR)
(74) Mandataire: Le Coupanec, Pascale A.M.P.
(86) Numéro de dépôt international: PCT/FR2000/001728
(87) Numéro de publication internationale: WO 2000/078990

(56) Documents cités:
- WO-A-90/14842
- WO-A-96/08580
- WO-A-99/38961
- WO-A-99/67428
- US-A- 5 436 131
- US-A- 5 786 177
- MELNICK L ET AL.: "An Escherichia coli expression assay andscreen for immunodeficiency virus protease variants with decreased susceptibility to indinavir" ANTIMICROBIAL AGENTS AND CHEMOTHERAPY, vol. 42, no. 12, 1998, pages 3256-3265, XP000901687
- VASUDEVACHARI M B ET AL.: "Emergence of protease inhibitor resistance mutations in human immunodeficiency virus type 1 isolates from patients and rapid screening procedure for their detection" ANTIMICROBIAL AGENTS AND CHEMOTHERAPY, vol. 40, no. 11, 1996, pages 2535-2541, XP000901688
- MASCHERA B ET AL.: "Anaysis of resistance to human immunodeficiency virus type 1 protease inhibitors by using matched bacterial expression and proviral infection vectors" JOURNAL OF VIROLOGY, vol. 69, no. 9, 1995, pages 5431-5436, XP002137320
- MARTINEZ-PICADO ET AL: "Replicative fitness of protease inhibitor-resistant mutants of human immunodeficiency virus type 1" JOURNAL OF VIROLOGY,US,THE AMERICAN SOCIETY FOR MICROBIOLOGY, vol. 73, no. 5, mai 1999 (1999-05), pages 3744-3752, XP002126128 ISSN: 0022-538X
- ROSé J.R. ET AL: 'Regulation of Autoproteolysis of the HIV-1 and HIV-2 Proteases with Engineered Amino Acid Substitutions' THE JOURNAL OF BIOLOGICAL CHEMISTRY vol. 268, no. 15, 05 Juin 1993, USA, pages 11939 - 11945

SEQUENCE LISTING
<110> BIO MERIEUX
   ASSISTANCE PUBLIQUE, HOPITAUX DE PARIS
<120> Search method for resistance to anti-proteases of a strain of the HIV 2 virus from a biological sample taken from a patient.
<130> BR34443/CR/HG/klp
<140> 00 949555.7
   <141> 2000-06-21
<150> FR 99 07855
   <151> 1999-06-21
<160> 52
<170> PatentIn version 3.1
<210> 1
   <211> 9
   <212> DNA
   <213> Listeria sequence
<400> 1
   ccaaaaata 9
<210> 2
   <211> 9
   <212> DNA
   <213> Listeria sequence
<400> 2
   ccaaaagta 9
<210> 3
   <211> 9
   <212> DNA
   <213> Listeria sequence
<400> 3
   cctaaaata 9
<210> 4
   <211> 9
   <212> DNA
   <213> Listeria sequence
<400> 4
   ccaagaata 9
<210> 5
   <211> 9
   <212> DNA
   <213> Listeria sequence
<400> 5
   ccaagagta 9
<210> 6
   <211> 9
   <212> DNA
   <213> Listeria sequence
<400> 6
   cctagaata 9
<210> 7
   <211> 9
   <212> DNA
   <213> Listeria sequence
<400> 7
   tttataaac 9
<210> 8
   <211> 9
   <212> DNA
   <213> Listeria sequence
<400> 8
   tttataaat 9
<210> 9
   <211> 9
   <212> DNA
   <213> Listeria sequence
<400> 9
   tttatgaac 9
<210> 10
   <211> 9
   <212> DNA
   <213> Listeria sequence
<400> 10
   tttatgaat 9
<210> 11
   <211> 9
   <212> DNA
   <213> Listeria sequence
<400> 11
   gaaataaaa 9
<210> 12
   <211> 9
   <212> DNA
   <213> Listeria sequence
<400> 12
   gaaatagaa 9
<210> 13
   <211> 9
   <212> DNA
   <213> Listeria sequence
<400> 13
   gaagtaaaa 9
<210> 14
   <211> 9
   <212> DNA
   <213> Listeria sequence
<400> 14
   gaagtagaa 9
<210> 15
   <211> 9
   <212> DNA
   <213> Listeria sequence
<400> 15
   aacatcttt 9
<210> 16
   <211> 9
   <212> DNA
   <213> Listeria sequence
<400> 16
   aacattttt 9
<210> 17
   <211> 9
   <212> DNA
   <213> Listeria sequence
<400> 17
   aacctcttt 9
<210> 18
   <211> 9
   <212> DNA
   <213> Listeria sequence
<400> 18
   attctgaca 9
<210> 19
   <211> 9
   <212> DNA
   <213> Listeria sequence
<400> 19
   atcctgaca 9
<210> 20
   <211> 9
   <212> DNA
   <213> Listeria sequence
<400> 20
   attctaaca 9
<210> 21
   <211> 9
   <212> DNA
   <213> Listeria sequence
<400> 21
   atcctaaca 9
<210> 22
   <211> 9
   <212> DNA
   <213> Listeria sequence
<400> 22
   attatgaca 9
<210> 23
   <211> 9
   <212> DNA
   <213> Listeria sequence
<400> 23
   atcatgaca 9
<210> 24
   <211> 9
   <212> DNA
   <213> Listeria sequence
<400> 24
   ccagtagtc 9
<210> 25
   <211> 9
   <212> DNA
   <213> Listeria sequence
<400> 25
   ccaatagtc 9
<210> 26
   <211> 9
   <212> DNA
   <213> Listeria sequence
<400> 26
   aaaatagta 9
<210> 27
   <211> 9
   <212> DNA
   <213> Listeria sequence
<400> 27
   aaagtagta 9
<210> 28
   <211> 9
   <212> DNA
   <213> Listeria sequence
<400> 28
   ccaatcaac 9
<210> 29
   <211> 9
   <212> DNA
   <213> Listeria sequence
<400> 29
   ccaataaac 9
<210> 30
   <211> 9
   <212> DNA
   <213> Listeria sequence
<400> 30
   ccaatgaac 9
<210> 31
   <211> 25
   <212> DNA
   <213> Listeria sequence
<400> 31
   attacactcc aagaatagta ggggg 25
<210> 32
   <211> 25
   <212> DNA
   <213> Listeria sequence
<400> 32
   attatagccc aagaatagta ggggg 25
<210> 33
   <211> 25
   <212> DNA
   <213> Listeria sequence
<400> 33
   attatagtcc aagaatagta ggggg 25
<210> 34
   <211> 25
   <212> DNA
   <213> Listeria sequence
<400> 34
   attatacccc aagaatagta ggggg 25
<210> 35
   <211> 25
   <212> DNA
   <213> Listeria sequence
<400> 35
   attatagtcc aagaatagta ggagg 25
<210> 36
   <211> 25
   <212> DNA
   <213> Listeria sequence
<400> 36
   attatacccc aagaatagta ggagg 25
<210> 37
   <211> 25
   <212> DNA
   <213> Listeria sequence
<400> 37
   tagggggatt tatgaacacc aaaga 25
<210> 38
   <211> 25
   <212> DNA
   <213> Listeria sequence
<400> 38
   tagggggatt catgaacacc aaaga 25
<210> 39
   <211> 25
   <212> DNA
   <213> Listeria sequence
<400> 39
   taggaggatt catgaacacc aaaga 25
<210> 40
   <211> 25
   <212> DNA
   <213> Listeria sequence
<400> 40
   taggagggtt catgaacacc aaaga 25
<210> 41
   <211> 25
   <212> DNA
   <213> Listeria sequence
<400> 41
   aaaatgtaga agtaaaagta ctaaa 25
<210> 42
   <211> 25
   <212> DNA
   <213> Listeria sequence
<400> 42
   aaaatataga agtaaaagta ctaaa 25
<210> 43
   <211> 25
   <212> DNA
   <213> Listeria sequence
<400> 43
   aagatgtaga agtaaaggta ctaaa 25
<210> 44
   <211> 25
   <212> DNA
   <213> Listeria sequence
<400> 44
   aaaatgtaga agtagaagtt ctaaa 25
<210> 45
   <211> 25
   <212> DNA
   <213> Listeria sequence
<400> 45
   aaaatgtaga agtagaagtc ctgga 25
<210> 46
   <211> 25
   <212> DNA
   <213> Listeria sequence
<400> 46
   aaagtgtaga agtaagagtg ctaaa 25
<210> 47
   <211> 25
   <212> DNA
   <213> Listeria sequence
<400> 47
   ccccaatcaa cctctttggc agaaa 25
<210> 48
   <211> 25
   <212> DNA
   <213> Listeria sequence
<400> 48
   gcagaaatat tatgacagcc ttagg 25
<210> 49
   <211> 25
   <212> DNA
   <213> Listeria sequence
<400> 49
   gcagaaatat tatggcaacc ttagg 25
<210> 50
   <211> 25
   <212> DNA
   <213> Listeria sequence
<400> 50
   gcagaaatgt tatgacagct ttagg 25
<210> 51
   <211> 25
   <212> DNA
   <213> Listeria sequence
<400> 51
   gcagaaatat catgacagcc ttggg 25
<210> 52
   <211> 23
   <212> DNA
   <213> Listeria sequence
<400> 52
   gcagaaacat tatgacagcc tta 23

## Description

L'invention concerne un procédé de recherche d'une résistance du virus VIH-2 à un traitement par une anti-protéase, chez un patient contaminé par le VIH-2, ainsi que des sondes nucléotidiques utilisables dans une telle recherche.

Deux virus sont la cause du syndrome d'immunodéficience acquise (sida) : VIH-1 et VIH-2. VIH-1 est présent dans le monde entier alors que VIH-2 est présent principalement en Afrique de l'Ouest.

Des traitements antiviraux efficaces sont largement utilisés depuis 1996 dans les pays développés où le virus présent est VIH-1. Le coût de ces traitements ne permet pas leur utilisation dans les pays en voie de développement où le VIH-2 est présent.

Les traitements anti-rétroviraux sont répartis en 3 classes : anti-protéase (Indinavir, Ritonavir, Saquinavir, Nelfinavir et Amprenavir), inhibiteurs nucléosidique, de transcriptase inverse, dite RT (Zidovudine, Didanosine, Zalcitabine, Lamivudine, Stavudine, Abacavir, FTC et Adefovir) et inhibiteurs non nucléosidiques de RT (Nevirapine, Delavirdine et Efavirenz). Ces traitements sont souvent effectués conjointement ; on parle alors de multithérapies.

Les anti-protéases induisent des mutations primaires qui confèrent un degré élevé de résistance mais altèrent la capacité de réplication du virus. Il est nécessaire pour le virus de sélectionner des mutations secondaires pour être à la fois résistant et capable de se répliquer activement. Par ailleurs, des mutations de la transcriptase inverse ont été décrites dans le cas d'utilisation conjointe d'inhibiteurs nucléosidique de la RT.

Pendant le traitement de l'infection à VIH-1, notamment si la concentration circulante des agents traitants est insuffisante, la réplication virale n'est pas suffisamment inhibée ou remonte au-dessus du seuil de détection des techniques de charge virale disponibles (la "charge virale" est la mesure de la quantité des génomes de virus circulants). Du fait du taux d'erreur élevé de la transcription inverse, cela se traduit par l'apparition de mutations dans les gènes qui sont la cible des traitements : la protéase et la transcriptase inverse. Certaines mutations entraînent l'apparition de résistances, à des degrés divers, aux antiviraux. On aboutit à des situations d'échec virologique chez 20 à 40 % des patients traités par les multithérapies mises en pratique actuellement.

La mise en évidence de virus résistants à une ou plusieurs molécules chez un patient avant traitement ou lors d'une réaugmentation de la charge virale permet de choisir la meilleure association de molécules pour traiter le VIH-1.

Par exemple, des procédés d'identification de mutant du virus VIH-1 résistant aux traitements par anti-protéases comprenant l'identification de mutations dans la séquence peptidique de la protéase du VIH-1 impliquée dans la résistance sont décrits dans WO96/08580, par Melnick *et al*. (Antimicrobial Agent and Chemotherapy, 1998, 42(12) : 3256-65) et Maschera *et al*. (Journal of Virology, 1995, 69(9) : 5431-6). Ces procédés se fondent sur la construction et le criblage d'une bibliothèque de variants de protéases du VIH-1 comprenant différentes mutations.

D'autres méthodes, fondées sur l'analyse de la sensibilité d'une enzyme de restriction, *Hin*d II, ont également été développées (Vasudevachari *et al*. (Anti Microbial Agent and Chemotherapy, 1996, 40(11) : 2535-41).

Ces méthodes ont permis l'identification de mutants par exemple en position 82 ou 90 présentant une résistance accrue aux inhibiteurs de protéases.

Il n'y a pas actuellement de données publiées concernant la présence de mutations dans le génome du VIH-2, dues à l'utilisation d'anti-protéases.

Les traitements par les agents anti-protéases, actifs pour VIH-1, le sont aussi pour VIH-2. Cependant, aucune méthode n'est disponible pour aider le clinicien à déterminer la résistance aux traitements par anti-protéases chez les patients contaminés par le VIH-2.

La séquence d'acides aminés de la protéase de VIH-2 est connue. Dans la présente demande, la numérotation des acides aminés de cette séquence peut être déduite de celle décrite dans Human Retroviruses and Aids, 1997, Los Alamos National Laboratory, Los Alamos, New Mexico, Chapitre II, pp. B10 et B11. Le premier acide aminé de la séquence de la protéase, qui est considéré comme la position 1 dans la présente demande, est la proline en position 86 de la polyprotéine PoL de la souche ROD.

On a maintenant découvert que les traitements aux anti-protéases sont susceptibles de faire apparaître des mutations aux positions 45, 54, 64, 84 et 90, ainsi qu'aux positions 10, 46 et 82 de la protéase de VIH-2, et que les souches virales mutées ainsi apparues sont généralement résistantes à l'un au moins des agents anti-protéases utilisés.

L'objet de la présente invention est donc un procédé de recherche d'une résistance éventuelle d'une souche virale VIH-2 à un traitement par un agent anti-protéase.

Dans une phase préliminaire de recherche, il s'agit d'un procédé dans lequel:
a) on recherche, selon les méthodes connues, la présence d'au moins une mutation à l'une des positions 45, 54, 64, 84 et 90 ou à l'une des positions 10, 46 et 82 de la séquence protéique de la protéase de ladite souche virale provenant d'un échantillon biologique prélevé sur un patient contaminé par VIH-2,
b) on sélectionne, parmi les mutations trouvées en a), celles qui, après clonage dans un virus VIH-2, n'empêchent pas le clone viral obtenu de se multiplier en culture en présence dudit agent anti-protéase, et
c) dans le cas où au moins une mutation est sélectionnée à l'étape b), on conclut à l'existence d'une résistance audit agent anti-protéase mentionné en b).

On sélectionne, parmi les mutations trouvées en a), celles qui, lorsqu'elles sont présentes dans un gène cloné dans un virus VIH-2, confèrent au clone viral la propriété de ne pas être affecté significativement dans sa capacité à se multiplier en présence dudit agent anti-protéase. Pour mettre en oeuvre l'étape b), on peut opérer comme indiqué ci-après. Les mutations à tester sont introduites individuellement dans un clone de virus par mutagénèse dirigée selon la méthode décrite dans l'article de Kemp et al, J. Virol., 72(6), p 5093-5098, 1998. Les clones ainsi obtenus sont mis en culture avec comme référence un clone sauvage de virus, c'est-à-dire non muté, en présence des différentes drogues anti-protéases susceptibles d'agir contre le virus VIH-2. Une mesure du CI₅₀, par exemple avec un test colorimétrique (voir publication de Kemp citée ci-dessus), permet de déterminer l'importance de la mutation (mineure ou majeure) vis-à-vis des différentes drogues testées. On peut ainsi sélectionner les mutations qui permettent au virus de se multiplier en présence d'un agent anti-protéase, ces mutations donnant naissance à des souches résistantes à cet anti-protéase.

Bien entendu, dans le cas prévu en c), on doit envisager le traitement du patient, dans le futur, avec un agent anti-protéase différent de celui pour lequel l'existence d'une résistance a été ainsi révélée chez le patient considéré.

Dans le cas où l'étape b) n'a pas permis de sélectionner une mutation trouvée à l'étape a), on peut conclure que la mutation considérée n'a pas induit une résistance du virus VIH-2 à l'agent anti-protéase testé, chez le patient considéré.

Il est évident que, lorsque l'étape b) a permis d'identifier une mutation qui engendre une résistance à un agent anti-protéase donné, il n'y a pas lieu de mettre en oeuvre ultérieurement l'étape b) du procédé décrit ci-dessus. Dans ce cas, l'étape a) suffit puisque, le lien entre la mutation et la résistance à l'agent anti-protéase ayant été établi, une fois pour toutes, il est possible de passer directement à l'étape c) et de conclure à l'existence d'une résistance à l'agent anti-protéase étudié.

L'invention concerne notamment un procédé de recherche d'une résistance éventuelle d'une souche virale VIH-2 à un traitement par un agent anti-protéase, dans lequel on recherche la présence d'au moins une mutation choisie parmi les mutations suivantes :
K 45 R, I 54 M, I 64 V, I 84 L et L 90 M, ou bien encore V 10 I, I 46 V et I 82 M,
dans la séquence protéique de la protéase de ladite souche virale, et dans lequel on conclut à l'existence de ladite résistance en cas de présence de ladite mutation ou desdites mutations.

La représentation conventionnelle utilisée dans la présente demande pour décrire une mutation est la suivante : le nombre indique la position sur la séquence d'acides aminés de la protéase de VIH-2. La lettre située à gauche du nombre indique l'acide aminé de la souche sauvage selon la classification internationale, avec le code à une lettre. La lettre située à droite du nombre indique l'acide aminé, selon la même classification, qui résulte de l'apparition d'une mutation.

Par "souche" sauvage, l'on entend une souche de virus n'ayant pas muté suite à un traitement avec une anti-protéase.

Pour rechercher une mutation de la séquence protéique de la protéase de la souche virale considérée, on opère de préférence en recherchant une mutation correspondante sur la séquence nucléotidique du gène de ladite protéase. La recherche de ces mutations peut se faire sur l'ADN ou l'ARN. Bien entendu, dans cette recherche d'une mutation dans la séquence protéique par recherche de mutation dans la séquence nucléotidique, on tiendra compte de la dégénérescence du code génétique, à savoir qu'un même acide aminé peut être codé par des codons différents. On peut effectuer cette recherche de mutation dans la séquence nucléotique selon les méthodes connues, en particulier par les techniques d'hybridation ou de séquençage.

Dans un premier mode de réalisation de l'invention, une méthode d'hybridation à l'aide de sondes spécifiques est mise en oeuvre pour la recherche de la ou des mutation(s).

Un mode d'exécution particulier utilisant une méthode d'hybridation consiste à obtenir un polynucléotide contenant tout ou partie du gène de la protéase, et comprenant la séquence d'intérêt correspondant à la région contenant la mutation à rechercher. Un tel polynucléotide peut être obtenu notamment par amplification enzymatique. La méthode utilisée comprend alors les étapes consistant à mettre en contact ledit polynucléotide avec une sonde nucléotidique, fixée ou susceptible de se fixer sur un support solide, et capable de s'hybrider spécifiquement avec un tel polynucléotide uniquement dans le cas où le polynucléotide comporte la mutation étudiée ; puis à révéler, selon les méthodes connues, la présence éventuelle du polynucléotide, fixé au support solide notamment par l'intermédiaire de la sonde de capture. Pour cela, on peut soumettre le support solide à une étape de lavage, après quoi on révèle la présence du polynucléotide, à l'état fixé sur le support, soit par une méthode physique, soit à l'aide d'un marqueur approprié.

La fixation de la sonde peut être réalisée directement par adsorption ou par covalence. La fixation peut aussi être réalisée indirectement par l'intermédiaire d'une réaction du type ligand/antiligand comme le couple biotine/streptavidine ou haptène/anticorps, l'antiligand étant fixé par exemple sur le support solide et le ligand étant fixé sur la sonde.

Le polynucléotide peut aussi être marqué pendant l'étape d'amplification enzymatique, par exemple en utilisant un nucléoside triphosphate marqué pour la réaction d'amplification. Le nucléotide marqué sera un désoxyribonucléotide dans les systèmes d'amplification générant un ADN, comme la PCR, ou un ribonucléotide dans les techniques d'amplification générant un ARN, comme les techniques TMA ou NASBA.

Le polynucléotide peut aussi être marqué après l'étape d'amplification, par exemple en hybridant une sonde marquée selon la technique d'hybridation sandwich décrite dans le document WO 91/19812.

Un mode particulier de marquage de polynucléotides est décrit dans la demande FR 98 07870 de la demanderesse.

En variante, on peut préparer le polynucléotide comprenant tout ou partie du gène de la protéase par amplification enzymatique, en procédant par élongation d'amorces comportant un ligand. Le polynucléotide obtenu, qui comporte donc le ligand, peut être fixé sur le support solide par interaction avec un antiligand correspondant. On met ensuite en contact le support solide, sur lequel est fixé le polynucléotide, avec au moins une sonde capable de se fixer sur le polynucléotide de façon spécifique, uniquement s'il contient la mutation étudiée ou recherchée. Dans le cas où cette mutation est présente, la sonde se trouve fixée au support solide par l'intermédiaire de l'hybride qu'elle forme avec le polynucléotide lui-même fixé. Il reste alors à révéler la présence de l'hybride ainsi formé, selon les méthodes connues.

Dans un autre mode d'exécution, on utilise une méthode d'hybridation comprenant les étapes consistant à amplifier enzymatiquement tout ou partie du gène de la protéase à l'aide d'amorces portant un ligand pour générer un polynucléotide comportant au moins un ligand, à fixer le polynucléotide sur un support solide par interaction avec un antiligand comme décrit précédemment, à mettre en contact ledit polynucléotide fixé avec au moins une sonde capable de s'hybrider spécifiquement avec celui-ci, et à révéler l'hybride éventuellement formé. La sonde ne doit s'hybrider que si le polynucléotide contient la mutation recherchée.

D'autres méthodes de détection par hybridation sont envisageables comme décrit dans Kricka et al., Clinical Chemistry, 45(4), pp. 453-458, 1999, ou Keller G.H. et al., DNA Probes, 2nd Ed., Stockton Press, 1993, sections 5 et 6, pp. 173-249.

Le terme "support solide" tel qu'utilisé ici inclut tous les matériaux sur lesquels peut être immobilisé un polynucléotide. Des matériaux de synthèse, ou des matériaux naturels, éventuellement modifiés chimiquement, peuvent être utilisés comme support solide, notamment les polysaccharides tels que les matériaux à base de cellulose, par exemple du papier, des dérivés de cellulose tels que l'acétate de cellulose et la nitrocellulose, ou le dextran ; des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels, etc. Le support solide peut être sous la forme d'une plaque de microtitration, d'une membrane comme décrit dans la demande WO 9412670, d'une particule ou d'une biopuce.

Par "biopuce", on entend un support solide de dimension réduite où sont fixés une multitude de polynucléotides à des positions prédéterminées.

Des exemples de ces biopuces sont donnés par exemple dans les publications de G. Ramsay. Nature Biotechnology, 16, pp. 40-44, 1998 : F. Ginot, Human Mutation, 10, pp. 1-10. 1997 ; J. Cheng et al, Molecular diagnosis, 1(3), p. 183-200, 1996 ; T. Livache et al, Nucleic Acids Research, 22(15), pp. 2915-2921, 1994 ; J. Cheng et al, Nature Biotechnology, 16, pp. 541-546, 1998. La propriété principale du support solide doit être de conserver les caractéristiques d'hybridation des sondes sur la cible et de permettre un bruit de fond minimum pour la méthode de détection. Un avantage des biopuces est qu'elles simplifient l'utilisation de nombreuses sondes tenant compte du polymorphisme du virus dans les zones voisines de la mutation à rechercher. Une biopuce permettant de vérifier la présence ou l'absence de mutations peut être réalisée en suivant la procédure décrite par Kozal M. et al. Nature Medicine, 2, pp. 753-759, 1996, en fonction des alignements des séquences connues pour les différentes souches du VIH-2.

Par "marqueur", on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la bêtagalactosidase, la glucose-6-phosphate déshydrogénase; les chromophores comme les composés fluorescents, luminescents ou colorants; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.

Des systèmes d'amplification du signal peuvent être utilisés comme décrit dans le document WO/95 08000 et dans ce cas, la réaction préliminaire d'amplification enzymatique peut ne pas être nécessaire.

Le terme "amorce" désigne une séquence oligonucléotidique capable de s'hybrider à une séquence nucléique d'intérêt et de servir de point de départ à une réaction d'élongation enzymatique pour produire un fragment d'acide nucléique complémentaire d'une cible d'intérêt, comme le gène de la protéase ou une partie de ce gène. L'amorce a une taille comprise par exemple entre 5 et 50 nucléotides, en particulier entre 10 et 30 nucléotides. De préférence, les amorces seront choisies dans des régions conservées du virus VIH-2 pour permettre l'amplification de toutes les souches du virus susceptibles d'être rencontrées chez un patient, et donc de faire face au polymorphisme inhérent au virus VIH-2.

Les sondes permettant de mettre en évidence une mutation sur les positions 45 et/ou 54 et/ou 64 et/ou 84 et/ou 90, ainsi que celles permettant de mettre en évidence une mutation sur les positions 10 et/ou 46 et/ou 82 en s'hybridant sur tout ou partie du gène de la protéase du virus VIH-2 présent dans un échantillon biologique, sont aussi un objet de la présente invention.

Le terme "sonde" fait référence à une séquence oligonucléotidique capable de s'hybrider spécifiquement à une séquence nucléique d'intérêt. En l'occurrence, le but de la présente invention étant de discriminer une mutation ponctuelle sur le gène de la protéase du VIH-2, la sonde doit permettre, dans les conditions d'hybridation ou de lavage prédéterminées, de différencier une mutation ponctuelle. La taille de ces sondes est comprise entre 5 et 40 nucléotides, et notamment entre 9 et 25 nucléotides. Des méthodes de détermination de ces sondes sont décrites par exemple dans la demande de brevet WO 97/27332. La sonde sera construite par exemple de telle manière que la position de la mutation à mettre en évidence soit sensiblement au centre de la sonde.

Les oligonucléotides utilisés comme amorces ou sondes peuvent comporter des nucléotides naturels ou modifiés comme les phosphorothioates, les H-phosphonates, les alkylphosphorothioates, ou des analogues de nucléotides contenant des bases comme l'inosine ou la nébularine à la place des bases puriques ou pyrimidiques présentes dans les nucléotides A, T, C, G, U.

Ces amorces ou sondes peuvent aussi être composées totalement ou partiellement de nucléosides d'anomérie alpha ou bêta ou d'isomères de la série D ou L, ou encore de PNA (Nielsen et al, Nuclèic Acid Research, 21(2), pp. 197-200, 1993).

Dans un autre mode de réalisation de l'invention, on recherche la ou les mutations par séquençage de tout ou partie du gène de la protéase.

Les différentes méthodes de séquençage sont bien connues : on peut utiliser notamment les méthodes de séquençage de Sanger, les méthodes de séquençage utilisant 4 puits pour la réaction des séquences étudiées avec des amorces de séquençage marquées par 4 fluorophores différents (Procédure « ABI Prism Dye Primer » de Perkin Elmer), ou le procédé décrit dans le brevet US 5795722 (Visible Genetics), ou bien la méthode utilisant non pas des amorces marquées mais des nucléotides marqués (procédure « ABI Prism Dye Terminator », Perkin Elmer). Les méthodes de séquençage sont décrites, par exemple, dans Molecular Cloning, A Laboratory Manual, Sambrook, Fritsch and Maniatis, Cold Spring Harbor Laboratory Press, 1989, Chapitre 13.

Dans un mode particulier de réalisation de l'invention, on recherche uniquement la présence d'une ou plusieurs mutations données. Dans un autre mode de réalisation de l'invention, on recherche à la fois la séquence nucléotidique mutée et la séquence nucléotidique sauvage (non mutée). Dans le cas où l'on met en oeuvre une méthode d'hybridation, on définit donc pour chaque position susceptible de muter au moins deux types de sondes : un type de sondes spécifique de la séquence mutée et un type de sondes spécifique de la séquence sauvage. L'utilisation de ces deux types de sondes permet de contrôler la méthode, puisque l'un au moins des deux types de sondes doit réagir. Un autre avantage est de mettre en évidence, le cas échéant, la présence à la fois de souches mutées et de souches sauvages chez un patient.

De préférence, l'acide nucléique cible est soumis à une réaction préliminaire d'amplification enzymatique pour augmenter la sensibilité du test, mais il est envisageable de détecter directement l'acide nucléique cible. Les articles de Lewis (1992. Genetic Engineering News, **12**, 1-9) d'une part, et d'Abramson et Myers (1993. Curr. Opin. Biotechnol., 4, 41-47), d'autre part, donnent des exemples d'amplification de cible. La technique d'amplification enzymatique est par exemple choisie parmi les techniques NASBA (Nucleic Acid Sequence Based Amplification), TMA (Transcription Mediated Amplification), RT-PCR (Reverse Transcriptase-Polymerase Chain Reaction), SDA (Strand Displacement Amplification) ou LCR (Ligase Chain Reaction).

La recherche des souches virales mutantes est effectuée à partir d'un échantillon biologique éventuellement prétraité. Par "prétraitement", on entend les différentes étapes de traitement de l'échantillon pour rendre accessible l'acide nucléique cible, c'est-à-dire le gène de la protéase, comme par exemple, la lyse, la fluidification, la concentration, la capture (voir par exemple US 5 750 338 et US 5 766 849) selon les méthodes connues en soi.

Pour extraire l'ARN viral, on peut utiliser par exemple le réactif vendu par la société Boeringher Mannheim (High Pure Viral RNA référence 1858882) ou le kit Quiagen (Viral RNA référence 29504). D'autres procédures sont décrites dans Maniatis et al., Molecular Cloning : A Laboratory Manual, 2^{e} Edition, Cold Spring Harbor Laboratory Press, 1989. L'échantillon biologique peut être un prélèvement quelconque du corps humain ou éventuellement un prélèvement enrichi par culture, comme par exemple, le sang, le sperme, un tissu cutané, un lavage broncho-alvéolaire, une biopsie, l'urine, des colonies, une culture liquide, etc.

Les exemples suivants illustrent l'invention.

### EXEMPLES

### EXEMPLE 1

Une étude a porté sur trois patients contaminés par le VIH-2. Les patients 1 et 2 n'avaient jamais reçu d'anti-protéases. Le patient 3 avait déjà reçu du Ritonavir pendant 8 mois, et ce traitement avait été arrêté 5 mois avant le début de l'étude. Le premier échantillon étudié a été prélevé avant le début du traitement, chez les patients 1 et 2, et 6 semaines après le début du traitement par Ritonavir chez le patient 3. Chez les patients 1 et 2, des échantillons prélevés respectivement 2 mois et 5 mois après le début du traitement ont été étudiés. Pour le patient 3, deux échantillons (8 mois et 11 mois) ont été analysés. Le traitement était constitué de Ritonavir pour le patient 2 et de Ritonavir/Saquinavir pour les patients 1 et 3, aux doses préconisées.

### Méthodes :

Les plasmas ont été obtenus par centrifugation du sang total à 800xg pendant 10 minutes et clarifiés par une deuxième centrifugation à 3 000 xg pendant 15 minutes.

500 microlitres de plasma pur ont été ajoutés à 1,5 millilitre de culture de lymphocytes frais stimulés par la PHA (10⁶ cellules / boîte). La réplication virale dans le surnageant a été suivie deux fois par semaine par la mesure de la concentration d'antigène P 24 de VIH (ELAVIA Ag I, Sanofi Diagnostics Pasteur). Les surnageants positifs ont été stockés à - 80° C. Après ultracentrifugation de 1 millilitre de surnageant (23-500xg pendant 1 heure), l'ARN de VIH-2 a été extrait à l'aide du kit Amplicor HCV Spécimen Préparation (Roche).

Le gène Protéase a été rétrotranscrit à partir de 10 microlitres de la solution d'ARN viral et amplifié avec le kit Titan One Tube RT-PCR (Roche Molecular Diagnostics). La transcription inverse et la première amplification ont été effectuées avec les amorces 3' Prot et 5' RT 3 (voir ci-dessous). La réaction à 50 °C pendant 30 minutes est suivie d'une étape de dénaturation à 94 °C pendant 5 minutes puis de 40 cycles (30 secondes à 94°C, 30 secondes à 55°C, 90 secondes à 68° C) et enfin à 68° C pendant 7 minutes. La deuxième étape de la PCR a été effectuée avec 5 microlitres du produit de la première étape avec les amorces 3' RTD et 5' Prot 2.1, avec une dénaturation de 5 minutes à 94° C, suivie de 30 cycles (30 secondes à 94° C, suivie de 30 cycles à 55 ° C et 30 secondes à 72° C) et enfin 10 minutes à 72° C. La séquence des amorces est la suivante :
3' Prot: CAGGGGCTGACACCAACAGCACCCCC
5' RT 3: CCATTTTTTCACAGATCTCTTTTAATGCCTC
3' RTD: ATGTGGGGGTATTATAAGGATTT
5' Prot 2.1: GAAAGAAGCCCCGCAACTTC
Les produits d'amplification ont été purifiés avec le kit QUIACQUICK PCR purification (Quiagen) et séquencées directement à l'aide des amorces 3 ' RTD et 5 ' Prot 2.1 à l'aide du kit ABI Prism Dye Terminator Cycle Sequencing (Applied Biosystem). Ils ont été analysés avec le séquenceur automatique Applied Biosystem 377 et les séquences ont été alignées avec les séquences consensus de VIH-2 (sous-types A et

### Résultats :

Avant traitement, aucune mutation n'a été détectée par rapport aux séquences consensus VIH-2 A et B.

Après traitement, on a observé les mutations suivantes :
- position 45 : chez les patients 1 et 2, on a observé la coexistence d'une population non-mutée (lysine ; codon AAA) et d'une population mutée (arginine ; codon AGA), c'est-à-dire la mutation K45K/R ;
- position 54 : on a observé chez les patients 1 et 2, le remplacement de l'isoleucine (codon ATA) par la méthionine (codon ATG) : soit la mutation I54M ;
- position 64 : on a observé une population non mutée et une population dans laquelle l'isoleucine (codon ATA) est remplacée par une valine (codon GTA) : soit la mutation 164I/V ;
- position 84 : chez le patient 3 on a observé une population non mutée (isoleucine ; codon ATC) et une population mutée avec remplacement de l'isoleucine par une leucine (codon CTC) : soit la mutation I84I/L ;
- position 90: chez les 3 patients, on a observé le remplacement de la leucine (codon CTG) par une méthionine (codon ATG) : soit la mutation L90M.

De façon analogue, on a observé les mutations suivantes :
- position 10 : le remplacement de la valine (codon : GTA) par une isoleucine (codon : ATA) : soit la mutation V 10 I, lors du traitement d'un patient par du Ritonavir ;
- position 46 : le remplacement de l'isoleucine (codon : ATA) par une valine (codon : GTA) : soit la mutation I 46 V, lors du traitement d'un patient par du Ritonavir ; et
- position 82 : le remplacement de l'isoleucine (codon : ATA) par une méthionine (codon : ATG) : soit la mutation I 82 M, lors du traitement d'un patient par de l'Indinavir.

### EXEMPLE 2 : Exemple de sondes utilisables pour détecter les mutations sur le gène de la protéase de VIH-2

Les sondes utilisables pour rechercher l'existence éventuelle de mutations, conformément à l'invention, selon les techniques d'hybridation, peuvent être définies à partir des alignements publiés par Myers G et al., 1997, Human Retroviruses and AIDS : A compilation and analysis of nucleic acid and aminoacid sequences, Los Alamos National Laboratory, Los Alamos ; New Mexico.

Bien entendu, outre les sondes définies expressément ci-dessous, l'invention comprend également les sondes nucléotidiques équivalentes, c'est-à-dire des sondes permettant de détecter les mêmes mutations, sur la séquence protéique de la protéase que celles détectées par les sondes définies ci-dessous, en tenant compte de la dégénérescence du code génétique, autrement dit en tenant compte du fait qu'un même acide aminé peut être codé par différents codons.

On entend donc par l'expression "séquences nucléotidiques équivalentes", toutes séquences nucléotidiques différant entre elles par au moins un nucléotide mais dont la traduction conduit à la même séquence protéique, en d'autres termes toutes les séquences nucléotidiques codant pour la même séquence protéique.

Evidemment, cette remarque est valable pour chacun des codons de chacune des sondes. Ainsi, par exemple l'acide aminé en position 53 de la protéase de HIV- 2 est une phénylalanine qui peut être codée soit par le codon TTT soit par le codon TTC. Les sondes correspondant à chacune de ces possibilités font bien entendu partie de l'invention.

En fonction des conditions particulières de l'hybridation, et notamment de la température et de la composition des tampons d'hybridation et de lavage, il est possible de définir des sondes qui devront comprendre au moins l'une des séquences minimums ci-dessous, ou leurs complémentaires. Des sondes contenant ces séquences permettent de discriminer les mutations dans un procédé d'hybridation. Bien entendu, des sondes analogues, obtenues notamment en introduisant des analogues de base, tels que l'inosine ou la nébularine, en des positions oùun polymorphisme dû à une variabilité intrinsèque du virus est présent, permettent d'obtenir un résultat similaire et font aussi partie de l'invention.

Ces séquences sont données dans le sens 5' vers 3' :
CCA AAA ATA pour une forme sauvage de la position 45.
CCA AAA GTA pour une forme sauvage de la position 45.
CCT AAA ATA pour une forme sauvage de la position 45.
CCA AGA ATA pour une forme mutée de la position 45.
CCA AGA GTA pour une forme mutée de la position 45.
CCT AGA ATA pour une forme mutée de la position 45.
TTT ATA AAC pour une forme sauvage de la position 54.
TTT ATA AAT pour une forme sauvage de la position 54.
TTT ATG AAC pour une forme mutée de la position 54.
TTT ATG AAT pour une forme mutée de la position 54.
GAA ATA AAA pour une forme sauvage de la position 64.
GAA ATA GAA pour une forme sauvage de la position 64.
GAA GTA AAA pour une forme mutée de la position 64.
GAA GTA GAA pour une forme mutée de la position 64.
AAC ATC TTT pour une forme sauvage de la position 84.
AAC ATT TTT pour une forme sauvage de la position 84.
AAC CTC TTT pour une forme mutée de la position 84.
ATT CTG ACA pour une forme sauvage de la position 90.
ATC CTG ACA pour une forme sauvage de la position 90.
ATT CTA ACA pour une forme sauvage de la position 90.
ATC CTA ACA pour une forme sauvage de la position 90.
ATT ATG ACA pour une forme mutée de la position 90.
ATC ATG ACA pour une forme mutée de la position 90.
ou bien encore :
CCA GTA GTC pour une forme sauvage de la position 10.
CCA ATA GTC pour une forme mutée de la position 10.
AAA ATA GTA pour une forme sauvage de la position 46.
AAA GTA GTA pour une forme mutée de la position 46.
CCA ATC AAC pour une forme sauvage de la position 82.
CCA ATA AAC pour une forme sauvage de la position 82.
CCA ATG AAC pour une forme mutée de la position 82.

Pour obtenir des sondes plus longues que celles ayant les séquences minimum de 9 nucléotides représentées ci-dessus, on doit bien entendu choisir les nucléotides supplémentaires de façon à respecter la séquence des régions adjacentes de part et d'autre de la séquence minimum dans le gène de la protéase d'une souche de VIH-2. Ces séquences peuvent être obtenues dans les banques de données.

Par exemple, on peut utiliser les sondes qui sont indiquées ci- après, ou leurs complémentaires.
(a) position 45 :
   On peut utiliser une sonde comportant par exemple 9 à 25 nucléotides (de préférence répartis symétriquement autour du codon muté AGA), dont la séquence est incluse dans l'une des séquences suivantes :
      ATTACACTCCAAGAATAGTAGGGGG
      ATTATAGCCCAAGAATAGTAGGGGG
      ATTATAGTCCAAGAATAGTAGGGGG
      ATTATACCCCAAGAATAGTAGGGGG
      ATTATAGTCCAAGAATAGTAGGAGG
      ATTATACCCCAAGAATAGTAGGAGG
(b) position 54 :
   On peut utiliser une sonde comportant par exemple 9 à 25 nucléotides (de préférence répartis symétriquement autour du codon muté ATG), dont la séquence est incluse dans l'une des séquences suivantes :
      TAGGGGGATTTATGAACACCAAAGA
      TAGGGGGATTCATGAACACCAAAGA
      TAGGAGGATTCATGAACACCAAAGA
      AGGAGGGTTCATGAACACCAAAGA
(c) position 64 :
   On peut utiliser une sonde comportant par exemple 9 à 25 nucléotides (de préférence répartis symétriquement autour du codon muté GTA), dont la séquence est incluse dans l'une des séquences suivantes :
      AAAATGTAGAAGTAAAAGTACTAAA
      AAAATATAGAAGTAAAAGTACTAAA
      AAGATGTAGAAGTAAAGGTACTAAA
      AAAATGTAGAAGTAGAAGTTCTAAA
      AAAATGTAGAAGTAGAAGTCCTGGA
      AAAGTGTAGAAGTAAGAGTGCTAAA
(d) position 84 :
   On peut utiliser une sonde comportant par exemple 9 à 25 nucléotides (de préférence répartis symétriquement autour du codon muté CTC), dont la séquence est incluse dans la séquence suivante :
      CCCCAATCAACCTCTTTGGCAGAAA
(e)position 90 :
   On peut utiliser une sonde comportant par exemple 9 à 25 nucléotides (de préférence répartis symétriquement autour du codon muté ATG), dont la séquence est incluse dans l'une des séquences suivantes :
      GCAGAAATATTATGACAGCCTTAGG
      GCAGAAATATTATGGCAACCTTAGG
      GCAGAAATGTTATGACAGCTTTAGG
      GCAGAAATATCATGACAGCCTTGGG
      GCAGAAACATTATGACAGCCTTA

## Revendications

1. Procédé de recherche, dans un échantillon biologique prélevé sur un patient contaminé par VIH-2 contenant au moins une souche virale de VIH-2, d'une résistance éventuelle de ladite souche virale VIH-2 à un traitement par un agent anti-protéase, dans lequel on recherche, selon les méthodes connues, la présence d'au moins une mutation à l'une des positions 45, 54, 64, 84 et 90 de la séquence protéique de la protéase de ladite souche virale, ladite mutation ayant été préalablement reconnue comme induisant ladite résistance, et dans lequel on conclut, dans le cas où une telle mutation a été trouvée, à l'existence d'une souche virale résistante audit agent anti-protéase chez le patient considéré.

2. Procédé selon la revendication 1, dans lequel :
a) on recherche, selon les méthodes connues, la présence d'au moins une mutation à l'une desdites positions de la séquence protéique de la protéase de ladite souche virale provenant d'un échantillon biologique prélevé sur un patient contaminé par VIH-2,
b) on sélectionne, parmi les mutations trouvées en a), celles qui, après clonage dans un virus VIH-2, n'empêchent pas le clone viral obtenu de se multiplier en culture en présence dudit agent anti-protéase, et
c) dans le cas où au moins une mutation est sélectionnée à l'étape b), on conclut à l'existence d'une résistance audit agent anti-protéase mentionné en b).

3. Procédé selon la revendication 1, dans lequel on recherche la présence d'au moins une mutation choisie parmi les mutations suivantes :
K 45 R, I 54 M, I 64 V, I 84 L, L 90 M,
dans la séquence protéique de la protéase de ladite souche virale, et dans lequel on conclut à la présence de ladite résistance en cas de présence de ladite mutation ou desdites mutations.

4. Procédé selon l'une quelconque des revendications précédentes, dans lequel, pour rechercher une mutation de la séquence protéique de la protéase, on effectue la recherche d'une mutation correspondante sur la séquence nucléotidique du gène de ladite protéase.

5. Procédé selon la revendication 4, dans lequel on effectue ladite recherche à l'aide des techniques d'hybridation, selon les méthodes connues.

6. Procédé selon la revendication 4, dans lequel on effectue ladite recherche à l'aide des techniques de séquençage, selon les méthodes connues.

7. Sonde nucléotidique utilisable dans le procédé de l'une quelconque des revendications 1 à 5, ladite sonde ayant au plus 40 nucléotides et comprenant comme séquence minimum une séquence choisie dans le groupe constitué par :
(a)
CCA AGA ATA pour une forme mutée de la position 45,
CCA AGA GTA pour une forme mutée de la position 45,
CCT AGA ATA pour une forme mutée de la position 45,
TTT ATG AAC pour une forme mutée de la position 54,
TTT ATG AAT pour une forme mutée de la position 54,
GAA GTA AAA pour une forme mutée de la position 64,
GAA GTA GAA pour une forme mutée de la position 64,
AAC CTC TTT pour une forme mutée de la position 84,
ATT ATG ACA pour une forme mutée de la position 90,
ATC ATG ACA pour une forme mutée de la position 90,
éventuellement complétée par la séquence nucléotidique d'une région adjacente du gène de ladite protéase, de part et d'autre de la séquence minimum,
(b) une séquence nucléotidique différente d'une séquence définie en (a) par au moins un nucléotide mais dont la traduction conduit à une même séquence protéique, et
(c) une séquence complémentaire d'une séquence définie en (a) ou en (b).

8. Sonde nucléotidique selon la revendication 7, **caractérisée en ce qu'**elle est choisie parmi les suivantes :
ATTACACTCCAAGAATAGTAGGGGG
ATTATAGCCCAAGAATAGTAGGGGG
ATTATAGTCCAAGAATAGTAGGGGG
ATTATACCCCAAGAATAGTAGGGGG
ATTATAGTCCAAGAATAGTAGGAGG
ATTATACCCCAAGAATAGTAGGAGG
TAGGGGGATTTATGAACACCAAAGA
TAGGGGGATTCATGAACACCAAAGA
TAGGAGGATTCATGAACACCAAAGA
TAGGAGGGTTCATGAACACCAAAGA
AAAATGTAGAAGTAAAAGTACTAAA
AAAATATAGAAGTAAAAGTACTAAA
AAGATGTAGAAGTAAAGGTACTAAA
AAAATGTAGAAGTAGAAGTTCTAAA
AAAATGTAGAAGTAGAAGTCCTGGA
AAAGTGTAGAAGTAAGAGTGCTAAA
CCCCAATCAACCTCTTTGGCAGAAA
GCAGAAATATTATGACAGCCTTAGG
GCAGAAATATTATGGCAACCTTAGG
GCAGAAATGTTATGACAGCTTTAGG
GCAGAAATATCATGACAGCCTTGGG
GCAGAAACATTATGACAGCCTTA
ou une séquence complémentaire.

9. Procédé de recherche, dans un échantillon biologique prélevé sur un patient contaminé par VIH-2 contenant au moins une souche virale de VIH-2, d'une résistance éventuelle de ladite souche virale VIH-2 à un traitement par un agent anti-protéase, dans lequel on recherche, selon les méthodes connues, la présence d'au moins une mutation à l'une des positions 10, 46 ou 82 de la séquence protéique de la protéase de ladite souche virale, ladite mutation ayant été préalablement reconnue comme induisant ladite résistance, et dans lequel on conclut, dans le cas où telle mutation a été trouvée, à la présence d'une souche virale résistante audit agent anti-protéase chez le patient considéré.

10. Procédé selon la revendication 9, dans lequel :
a) on recherche, selon les méthodes connues, la présence d'au moins une mutation à l'une desdites positions de la séquence protéique de la protéase de ladite souche virale provenant d'un échantillon biologique prélevé sur un patient contaminé par VIH-2,
b) on sélectionne, parmi les mutations trouvées en a), celles qui, après clonage dans un virus VIH-2, n'empêchent pas le clone viral obtenu de se multiplier en culture en présence dudit agent anti-protéase, et
c) dans le cas où au moins une mutation est sélectionnée à l'étape b), on conclut à l'existence d'une résistance audit agent anti-protéase mentionné en b).

11. Procédé selon la revendication 9, dans lequel on recherche la présence d'au moins une mutation choisie parmi les mutations suivantes :
V 10 I, I 46 V et I 82 M,
dans la séquence protéique de la protéase de ladite souche virale, et dans lequel on conclut à la présence de ladite résistance en cas de présence de ladite mutation ou desdites mutations.

12. Procédé selon l'une quelconque des revendications 9 à 11, dans lequel, pour rechercher une mutation de la séquence protéique de la protéase, on effectue la recherche d'une mutation correspondante sur la séquence nucléotidique du gène de la protéase.

13. Procédé selon la revendication 12, dans lequel on effectue ladite recherche à l'aide des techniques de séquençage, selon les méthodes connues.

14. Procédé selon la revendication 12, dans lequel on effectue ladite recherche à l'aide des techniques d'hybridation, selon les méthodes connues.

15. Sonde nucléotidique utilisable dans le procédé de la revendication 14, ladite sonde ayant au plus 40 nucléotides et comprenant comme séquence minimum une séquence choisie dans le groupe constitué par :
(a)
CCA ATA GTC pour une forme mutée de la position 10,
AAA GTA GTA pour une forme mutée de la position 46,
CCA ATG AAC pour une forme mutée de la position 82,
éventuellement complétée par la séquence nucléotidique d'une région adjacente du gène de ladite protéase, de part et d'autre de la séquence minimum,
(b) une séquence nucléotidique différente de celle définie en (a), par au moins un nucléotide mais dont la traduction conduit à une même séquence protéique, et
(c) une séquence complémentaire d'une séquence définie en (a) ou en (b).

16. Utilisation d'une sonde nucléotidique pour rechercher dans un échantillon biologique prélevé sur un patient contaminé par VIH-2 contenant au moins une souche virale de VIH-2, la présence d'au moins une mutation à l'une des positions 45, 54, 64, 84 et 90 de la séquence protéique de la protéase de ladite souche virale, ladite mutation ayant été préalablement reconnue comme induisant une résistance de ladite souche virale VIH-2 à un traitement par un agent antiprotéase, ladite sonde ayant au plus 40 nucléotides et comprenant comme séquence minimum une séquence choisie dans le groupe constitué par :
(a)
CCA AGA ATA pour une forme mutée de la position 45,
CCA AGA GTA pour une forme mutée de la position 45,
CCT AGA ATA pour une forme mutée de la position 45,
TTT ATG AAC pour une forme mutée de la position 54,
TTT ATG AAT pour une forme mutée de la position 54,
GAA GTA AAA pour une forme mutée de la position 64,
GAA GTA GAA pour une forme mutée de la position 64,
AAC CTC TTT pour une forme mutée de la position 84,
ATT ATG ACA pour une forme mutée de la position 90,
ATC ATG ACA pour une forme mutée de la position 90,
éventuellement complétée par la séquence nucléotidique d'une région adjacente du gène de ladite protéase, de part et d'autre de la séquence minimum, et
(b) une séquence complémentaire d'une séquence définie en (a).

17. Utilisation d'une sonde nucléotidique pour rechercher dans un échantillon biologique prélevé sur un patient contaminé par VIH-2 contenant au moins une souche virale de VIH-2, la présence d'au moins une mutation choisie parmi les mutations suivantes :
V 10 I, I 46 V et I 82 M
dans la séquence protéique de la protéase de ladite souche virale, ladite mutation ayant été préalablement reconnue comme induisant une résistance de ladite souche virale VIH-2 à un traitement par un agent anti-protéase, ladite sonde ayant au plus 40 nucléotides et comprenant comme séquence minimum une séquence choisie dans le groupe constitué par :
(a)
CCA ATA GTC pour une forme mutée de la position 10,
AAA GTA GTA pour une forme mutée de la position 46,
CCA ATG AAC pour une forme mutée de la position 82,
éventuellement complétée par la séquence nucléotidique d'une région adjacente du gène de ladite protéase, de part et d'autre de la séquence minimum, et
(b) une séquence complémentaire d'une séquence en (a).

## Claims

1. Method for testing, in a biological sample from a patient infected by HIV-2 containing at least one HIV-2 viral strain, the resistance of said HIV-2 viral strain to treatment with an antiprotease agent, wherein, using known methods, the presence of at least one mutation at one of positions 45, 54, 64, 84, and 90 of the protein sequence of the protease of said viral strain is investigated, said mutation having previously been found to elicit said resistance, and wherein, if such a mutation is found, it is concluded that a viral strain resistant to said antiprotease agent is present in the patient in question.

2. Method according to Claim 1, wherein:
a) using known methods, the presence of at least one mutation at one of said positions of the protein sequence of the protease of said viral strain in a biological sample taken from a patient infected with HIV-2 is investigated,
b) of the mutations founds in a), those which, after cloning in an HIV-2 virus, do not prevent the virus clone obtained from multiplying in culture in the presence of said antiprotease drug are selected, and
c) if at least one mutation is selected at step b), it is concluded that resistance exists to the antiprotease drug referred to in b).

3. Method according to Claim 1, wherein the presence of at least one mutation chosen from the following mutations:
K 45 R, I 54 M, I 64 V, I 84 L and L 90 M,
in the protein sequence of the protease of said viral strain is investigated and in which said resistance is concluded to exist if said mutation or said mutations is or are present.

4. Method according to any of the foregoing claims, wherein, to detect a mutation of the protein sequence of the protease, a corresponding mutation is sought in the nucleotide sequence of the gene of said protease.

5. Method according to Claim 4, wherein said test is carried out using hybridization techniques, according to known methods.

6. Method according to Claim 4, wherein said test is carried out using sequencing techniques, according to known methods.

7. Nucleotide probe usable in the method according to any one of Claims 1 to 5, comprising, said probe having at most 40 nucleotides and comprising as a minimum sequence, a sequence chosen from the group comprised of:
CCA AGA ATA for a mutated form of position 45.
CCA AGA GTA for a mutated form of position 45.
CCT AGA ATA for a mutated form of position 45.
TTT ATG AAC for a mutated form of position 54.
TTT ATG AAT for a mutated form of position 54.
GAA GTA AAA for a mutated form of position 64.
GAA GTA GAA for a mutated form of position 64.
AAC CTC TTT for a mutated form of position 84.
ATT ATG ACA for a mutated form of position 90.
ATC ATG ACA for a mutated form of position 90,
possibly supplemented by the nucleotide sequence of an adjacent region of the gene of said protease, on either side of the minimum sequence,
(b) a nucleotide sequence different from a sequence defined in (a) by at least one nucleotide but whose translation leads to the same protein sequence, and
(c) a sequence complementary to a sequence defined in (a) or in (b).

8. Nucleotide probe according to claim 7, wherein it is chosen from the following:
ATTACACTCCAAGAATAGTAGGGGG
ATTATAGCCCAAGAATAGTAGGGGG
ATTATAGTCCAAGAATAGTAGGGGG
ATTATACCCCAAGAATAGTAGGGGG
ATTATAGTCCAAGAATAGTAGGAGG
ATTATACCCCAAGAATAGTAGGAGG
TAGGGGGATTTATGAACACCAAAGA
TAGGGGGATTCATGAACACCAAAGA
TAGGAGGATTCATGAACACCAAAGA
TAGGAGGGTTCATGAACACCAAAGA
AAAATGTAGAAGTAAAAGTACTAAA
AAAATATAGAAGTAAAAGTACTAAA
AAGATGTAGAAGTAAAGGTACTAAA
AAAATGTAGAAGTAGAAGTTCTAAA
AAAATGTAGAAGTAGAAGTCCTGGA
AAAGTGTAGAAGTAAGAGTGCTAAA
CCCCAATCAACCTCTTTGGCAGAAA
GCAGAAATATTATGACAGCCTTAGG
GCAGAAATATTATGGCAACCTTAGG
GCAGAAATGTTATGACAGCTTTAGG
GCAGAAATATCATGACAGCCTTGGG
GCAGAAACATTATGACAGCCTTA
or a sequence complementary.

9. Method for testing, in a biological sample from a patient infected by HIV-2 containing at least one HIV-2 viral strain, the resistance of the HIV-2 viral strain to treatment with an antiprotease agent, wherein, using known methods, the presence of at least one mutation in position 10 or 46 or 82 of the protein sequence of the protease of said viral strain is investigated, said mutation having previously been found to elicit said resistance, and wherein, if such a mutation is found, it is concluded that a viral strain resistant to said antiprotease agent is present in the patient in question.

10. Method according to Claim 9, wherein:
a) using known methods, the presence of at least one mutation at one of said positions of the protein sequence of the protease of said viral strain in a biological sample taken from a patient infected with HIV-2 is investigated,
b) of the mutations founds in a), those which, after cloning in an HIV-2 virus, do not prevent the virus clone obtained from multiplying in culture in the presence of said antiprotease drug are selected, and
c) if at least one mutation is selected at step b), it is concluded that resistance exists to the antiprotease drug referred to in b).

11. Method according to Claim 9, wherein the presence of at least one mutation chosen from the following mutations:
V 10 I, I 46 V, and I 82 M
in the protein sequence of the protease of said viral strain is investigated and in which said resistance is concluded to exist if said mutation or said mutations is or are present.

12. Method according to any of Claims 9 to 11, wherein, to detect a mutation of the protein sequence of the protease, a corresponding mutation is sought in the nucleotide sequence of the gene of said protease.

13. Method according to Claim 12, wherein said test is carried out using sequencing techniques, according to known methods.

14. Method according to Claim 12, wherein said test is carried out using hybridization techniques, according to known methods.

15. Nucleotide probe usable in the method according to Claim 14, said probe having at most 40 nucleotides and comprising, as a minimum sequence, a sequence chosen from the group comprised of:
(a)
CCA ATA GTC for a mutated form of position 10.
AAA GTA GTA for a mutated form of position 46.
CCA ATG AAC for a mutated form of position 82.
possibly supplemented by the nucleotide sequence of an adjacent region of the gene of said protease, on either side of the minimum sequence,
(b) a nucleotide sequence different from a sequence defined in (a) by at least one nucleotide but whose translation leads to the same protein sequence, and
(c) a sequence complementary to a sequence defined in (a) or in (b).

16. Use of nucleotide probe for testing, in a biological sample from a patient infected by HIV-2 containing at least one HIV-2 viral strain, the presence of at least one mutation in one of the positions 45, 54, 64, 84 and 90 of the protein sequence of the protease of said viral strain, said mutation having previously been found to elicit said resistance to said HIV-2 viral stain to a treatment with an antiprotease drug, said probe having at most 40 nucleotides and comprising as a minimum sequence, a sequence chosen from the group comprised of:
CCA AGA ATA for a mutated form of position 45.
CCA AGA GTA for a mutated form of position 45.
CCT AGA ATA for a mutated form of position 45.
TTT ATG AAC for a mutated form of position 54.
TTT ATG AAT for a mutated form of position 54.
GAA GTA AAA for a mutated form of position 64.
GAA GTA GAA for a mutated form of position 64.
AAC CTC TTT for a mutated form of position 84.
ATT ATG ACA for a mutated form of position 90.
ATC ATG ACA for a mutated form of position 90,
possibly supplemented by the nucleotide sequence of an adjacent region of the gene of said protease, on either side of the minimum sequence, and,
b) a sequence complementary to a sequence defined in (a).

17. Use of nucleotide probe for testing, in a biological sample from a patient infected by HIV-2 containing at least one HIV-2 viral strain, the presence of at least one mutation chosen from the following mutations:
V 10 I, I 46 V and I 82 M
in the protein sequence of the protease of said viral strain, said mutation having previously been found to elicit said resistance to said HIV-2 viral stain to a treatment with an antiprotease drug, said probe having at most 40 nucleotides and comprising as a minimum sequence, a sequence chosen from the group comprised of:
(a)
CCA ATA GTC for a mutated form of position 10.
AAA GTA GTA for a mutated form of position 46.
CCA ATG AAC for a mutated form of position 82.
possibly supplemented by the nucleotide sequence of an adjacent region of the gene of said protease, on either side of the minimum sequence, and,
(b) a sequence complementary to a sequence defined in (a).

## Patentansprüche

1. Verfahren zur Untersuchung, in einer biologischen Probe, die einem mit HIV-2 kontaminierten Patienten entnommen wurde, die mindestens einen HIV-2-Virusstamm enthält, einer möglichen Resistenz des HIV-2-Virusstammes gegenüber einer Behandlung durch ein proteasehemmendes Mittel, wobei, nach den bekannten Verfahren, die Gegenwart von mindestens einer Mutation an einer der Positionen 45, 54, 64, 84 und 90 der Proteinsequenz der Protease des Virusstammes untersucht wird, wobei die Mutation zuvor als Resistenz auslösend bekannt war und wobei im Fall der Feststellung einer solchen Mutation auf die Existenz eines Virusstammes, der gegenüber dem proteasehemmenden Mittel resistent ist, in dem betrachteten Patienten geschlossen wird.

2. Verfahren nach Anspruch 1, wobei:
a) nach dem bekannten Verfahren die Gegenwart von mindestens einer Mutation an einer der Positionen der Proteinsequenz der Protease des Virusstammes untersucht wird, der einer biologischen Probe entstammt, die einem mit HIV-2 kontaminierten Patienten entnommen wurde,
b) unter den unter a) festgestellten Mutationen diejenigen selektioniert werden, die nach Klonierung in ein HIV-2-Virus den erhaltenen viralen Klon nicht an der Vermehrung in Kultur in Gegenwart des proteasehemmenden Mittels hindern, und
c) im Fall, wobei mindestens eine Mutation in Schritt b) selektioniert wird, auf die Existenz einer Resistenz gegenüber dem unter b) erwähnten proteasehemmenden Mittel geschlossen wird.

3. Verfahren nach Anspruch 1, wobei die Gegenwart von mindestens einer Mutation, ausgewählt aus den folgenden Mutationen:
K 45 R, I 54 M, I 64 V, I 84 L, L 90 M,
in der Proteinsequenz der Protease des Virusstammes untersucht wird und wobei auf die Gegenwart der Resistenz im Fall der Gegenwart der Mutation oder den Mutationen geschlossen wird.

4. Verfahren nach einem der vorhergehenden Ansprüche, wobei zur Untersuchung einer Mutation der Proteinsequenz der Protease die Untersuchung einer entsprechenden Mutation in der Nucleotidsequenz des Gens der Protease durchgeführt wird.

5. Verfahren nach Anspruch 4, wobei die Untersuchung mit Hilfe von Hybridisierungstechniken nach den bekannten Verfahren durchgeführt wird.

6. Verfahren nach Anspruch 4, wobei die Untersuchung mit Hilfe von Sequenzierungstechniken nach den bekannten Verfahren durchgeführt wird.

7. Nucleotidsonde, die bei dem Verfahren nach einem der Ansprüche 1 bis 5 verwendbar ist, wobei die Sonde höchstens 40 Nucleotide aufweist und als minimale Sequenz eine Sequenz umfasst, ausgewählt aus der Gruppe bestehend aus:
(a)
CCA AGA ATA für eine mutierte Form der Position 45,
CCA AGA GTA für eine mutierte Form der Position 45,
CCT AGA ATA für eine mutierte Form der Position 45,
TTT ATG AAC für eine mutierte Form der Position 54,
TTT ATG AAT für eine mutierte Form der Position 54,
GAA GTA AAA für eine mutierte Form der Position 64,
GAA GTA GAA für eine mutierte Form der Position 64,
AAC CTC TTT für eine mutierte Form der Position 84,
ATT ATG ACA für eine mutierte Form der Position 90,
ATC ATG ACA für eine mutierte Form der Position 90,
gegebenenfalls vervollständigt mit einer Nucleotidsequenz einer von dem Gen der Protease benachbarten Region auf beiden Seiten der minimalen Sequenz,
(b) einer Nucleotidsequenz, die von der unter a) definierten Sequenz durch mindestens ein Nucleotid verschieden ist, deren Translation zu einer gleichen Proteinsequenz führt, und
(c) einer zu einer unter (a) oder unter (b) definierten Sequenz komplementären Sequenz.

8. Nucleotidsonde nach Anspruch 7, **dadurch gekennzeichnet, dass** sie unter den folgenden Sequenzen:
ATTACACTCCAAGAATAGTAGGGGG
ATTATAGCCCAAGAATAGTAGGGGG
ATTATAGTCCAAGAATAGTAGGGGG
ATTATACCCCAAGAATAGTAGGGGG
ATTATAGTCCAAGAATAGTAGGAGG
ATTATACCCCAAGAATAGTAGGAGG
TAGGGGGATTTATGAACACCAAAGA
TAGGGGGATTCATGAACACCAAAGA
TAGGAGGATTCATGAACACCAAAGA
TAGGAGGGTTCATGAACACCAAAGA
AAAATGTAGAAGTAAAAGTACTAAA
AAAATATAGAAGTAAAAGTACTAAA
AAGATGTAGAAGTAAAGGTACTAAA
AAAATGTAGAAGTAGAAGTTCTAAA
AAAATGTAGAAGTAGAAGTCCTGGA
AAAGTGTAGAAGTAAGAGTGCTAAA
CCCCAATCAACCTCTTTGGCAGAAA
GCAGAAATATTATGACAGCCTTAGG
GCAGAAATATTATGGCAACCTTAGG
GCAGAAATGTTATGACAGCTTTAGG
GCAGAAATATCATGACAGCCTTGGG
GCAGAAACATTATGACAGCCTTA
oder einer komplementären Sequenz ausgewählt ist.

9. Verfahren zur Untersuchung, in einer biologischen Probe, die einem mit HIV-2 kontaminierten Patienten entnommen wurde, die mindestens einen HIV-2-Virusstamm enthält, einer möglichen Resistenz des HIV-2-Virusstammes gegenüber einer Behandlung durch ein proteasehemmendes Mittel, wobei, nach den bekannten Verfahren, die Gegenwart von mindestens einer Mutation an einer der Positionen 10, 46 oder 82 der Proteinsequenz der Protease des Virusstammes untersucht wird, wobei die Mutation zuvor als Resistenz auslösend bekannt war und wobei im Fall der Feststellung einer solchen Mutation auf die Gegenwart eines Virusstammes, der gegenüber dem proteasehemmenden Mittel resistent ist, in dem betrachteten Patienten geschlossen wird.

10. Verfahren nach Anspruch 9, wobei:
(a) nach dem bekannten Verfahren die Gegenwart von mindestens einer Mutation an einer der Positionen der Proteinsequenz der Protease des Virusstammes untersucht wird, der einer biologischen Probe entstammt, die einem mit HIV-2 kontaminierten Patienten entnommen wurde,
(b) unter den unter a) festgestellten Mutationen diejenigen selektionert werden, die nach Klonierung in ein HIV-2-Virus den erhaltenen viralen Klon nicht an der Vermehrung in Kultur in Gegenwart des proteasehemmenden Mittels hindern, und
(c) im Fall, wobei mindestens eine Mutation in Schritt b) selektioniert wird, auf die Existenz einer Resistenz gegenüber dem unter b) erwähnten proteasehemmenden Mittel geschlossen wird.

11. Verfahren nach Anspruch 9, wobei die Gegenwart von mindestens einer Mutation, die aus den folgenden Mutationen ausgewählt ist:
V 10 I, I 46 V und I 82 M
in der Proteinsequenz der Protease des Virusstammes untersucht wird und wobei im Fall des Vorliegens der Mutation oder Mutationen auf die Gegenwart der Resistenz geschlossen wird.

12. Verfahren nach einem der Ansprüche 9 bis 11, wobei zur Untersuchung einer Mutation der Proteinsequenz der Protease die Untersuchung einer entsprechenden Mutation in der Nucleotidsequenz des Gens der Protease durchgeführt wird.

13. Verfahren nach Anspruch 12, wobei die Untersuchung mit Hilfe von Sequenzierungstechniken nach den bekannten Verfahren durchgeführt wird.

14. Verfahren nach Anspruch 12, wobei die Untersuchung mit Hilfe von Hybridisierungstechniken nach den bekannten Verfahren durchgeführt wird.

15. Nucleotidsonde, die bei dem Verfahren nach Anspruch 14 verwendbar ist, wobei die Sonde höchstens 40 Nucleotide aufweist und als minimale Sequenz eine Sequenz umfasst, ausgewählt aus der Gruppe bestehend aus:
(a)
CCA ATA GTC für eine mutierte Form der Position 10,
AAA GTA GTA für eine mutierte Form der Position 46,
CCA ATG AAC für eine mutierte Form der Position 82,
gegebenenfalls vervollständigt mit einer Nucleotidsequenz einer von dem Gen der Protease benachbarten Region auf beiden Seiten der minimalen Sequenz,
(b) einer Nucleotidsequenz, die von der unter a) definierten Sequenz durch mindestens ein Nucleotid verschieden ist, deren Translation zu einer gleichen Proteinsequenz führt, und
(c) einer zu einer unter (a) oder unter (b) definierten Sequenz komplementären Sequenz.

16. Verwendung einer Nucleotidsonde zur Untersuchung in einer biologischen Probe, die einem mit HIV-2 kontaminierten Patienten entnommen wurde, die mindestens einen HIV-2-Virusstamm enthält, des Vorliegens von mindestens einer Mutation an einer der Positionen 45, 54, 64, 84 und 90 der Proteinsequenz der Protease des Virusstammes, wobei die Mutation zuvor als eine Resistenz des HIV-2-Virusstammes gegenüber einer Behandlung durch ein proteasehemmendes Mittel auslösend bekannt war, wobei die Sonde höchstens 40 Nucleotide aufweist und als minimale Sequenz eine Sequenz umfasst, ausgewählt aus der Gruppe bestehend aus:
(a)
CCA AGA ATA für eine mutierte Form der Position 45,
CCA AGA GTA für eine mutierte Form der Position 45,
CCT AGA ATA für eine mutierte Form der Position 45,
TTT ATG AAC für eine mutierte Form der Position 54,
TTT ATG AAT für eine mutierte Form der Position 54,
GAA GTA AAA für eine mutierte Form der Position 64,
GAA GTA GAA für eine mutierte Form der Position 64,
AAC CTC TTT für eine mutierte Form der Position 84,
ATT ATG ACA für eine mutierte Form der Position 90,
ATC ATG ACA für eine mutierte Form der Position 90,
gegebenenfalls mit einer Nucleotidsequenz einer von dem Gen der Protease benachbarten Region auf beiden Seiten der minimalen Sequenz vervollständigt und
(b) einer zu einer unter (a) definierten Sequenz komplementären Sequenz.

17. Verwendung einer Nucleotidsonde zur Untersuchung in einer biologischen Probe, die einem mit HIV-2 kontaminierten Patienten entnommen wurde, die mindestens einen HIV-2-Virusstamm enthält, des Vorliegens von mindestens einer Mutation, ausgewählt unter den folgenden Mutationen:
V 10 I, I 46 V, und I 82 M
in der Proteinsequenz der Protease des Virusstammes, wobei die Mutation zuvor als eine Resistenz des HIV-2-Virusstammes gegenüber einer Behandlung durch ein proteasehemmendes Mittel auslösend bekannt war, wobei die Sonde höchstens 40 Nucleotide aufweist und als minimale Sequenz eine Sequenz umfasst, ausgewählt aus der Gruppe bestehend aus:
(a)
CCA ATA GTC für eine mutierte Form der Position 10,
AAA GTA GTA für eine mutierte Form der Position 46,
CCA ATG AAC für eine mutierte Form der Position 82,
gegebenenfalls vervollständigt mit einer Nucleotidsequenz einer von dem Gen der Protease benachbarten Region auf beiden Seiten der minimalen Sequenz, und
(b) einer zu einer Sequenz unter (a) komplementären Sequenz.
